# FASCICULE DE BREVET EUROPEEN

(11) **EP 1 409 457 B1**
(45) Date de publication et mention de la délivrance du brevet: **02.08.2006**
(21) Numéro de dépôt: 02770025.1
(22) Date de dépôt: 15.07.2002
(51) Int. Cl.: C07D 211/58

(54) **PROCEDE POUR LA PREPARATION DE LA 4-METHYLAMINO-4-PHENYLPIPERIDINE**
VERFAHREN ZUR HERSTELLUNG VON 4 METHYLAMINO 4 PHENYLPIPERIDIN
METHOD FOR PREPARING 4-METHYLAMINO-4-PHENYLPIPERIDINE

(30) Priorité: 16.07.2001 FR 0109575
(43) Date de publication de la demande: 21.04.2004
(73) Titulaire: Sanofi-Aventis, 75013 Paris (FR)
(72) Inventeur: ANNE-ARCHARD, Gilles, F-31500 Toulouse (FR); GROS-CLAUDE, Patrick, F-31450 Berberaud (FR)
(74) Mandataire: Varady, Peter
(86) Numéro de dépôt international: PCT/FR2002/002498
(87) Numéro de publication internationale: WO 2003/008381

(56) Documents cités:
- WILSON W: "SYNTHETIC ANALGESICS AND RELATED COMPOUNDS PART I. AMIDINES AND 4: 5-DIHYDROGLYOXALINES" JOURNAL OF THE CHEMICAL SOCIETY, CHEMICAL SOCIETY, LONDON, GB, 1950, pages 2173-2176, XP001062330 ISSN: 0368-1769
- KALIR, PELAH: "1-Phenylcycloalkylamine Derivatives I" ISRAEL JOURNAL OF CHEMISTRY., XP001075065 XX, XX ISSN: 0021-2148

## Description

La présente invention concerne un procédé pour la préparation de la 4-méthylamino-4-phénylpipéridine et de ses sels. La 4-méthylamino-4-phénylpipéridine est un intermédiaire utile pour la préparation de principes actifs à usage thérapeutique, par exemple des antagonistes des tachylcinines.

Selon G.A.M Giardina et al. (Bioorg. Med. Chem. Letters, 1996, 6, 2307-2310) la 4-méthylamino-4-phénylpipéridine peut être préparée en six étapes à partir de la 1-benzyl-4-pipéridone par réaction avec le phényllithium, traitement du 1-benzyl-4-phenylpipéridin-4-ol ainsi obtenu avec l'acide sulfurique et l'acide acétique dans l'acétonitrile, désacétylation de la 4-acétamido-1-benzyl-4-phénylpipéridine, formylation de la 4-amino-1-benzyl-4-phénylpipéridine, réduction du dérivé N-formylé ainsi obtenu avec de l'hydrure de lithium et d'aluminium et débenzylation finale de la 1-benzyl-4-méthylamino-4-phénylpipéridine par hydrogénation catalytique. Bien que ces six étapes se déroulent avec des bons rendements, le procédé présente des inconvénients qui en rendent peu aisée l'application industrielle. Plus particulièrement, dans la préparation de la 4-méthylamino-4-phénylpipéridine deux des six étapes sont conduites à reflux pendant trois jours, ce qui allonge la durée du procédé de façon considérable. De plus cette préparation comporte, dans la première étape, l'utilisation du phényllithium qui peut entraîner des problèmes non négligeables au niveau industriel.

Il a été maintenant trouvé qu'à partir d'un produit commercial facilement accessible, la 1-benzyl-4-cyano-4-phénylpipéridine, il est possible d'obtenir une 4-alcoxycarbonylamino-1-benzyl-4-phénylpipéridine en deux seules étapes qui peuvent être conduites dans le même réacteur (en anglais "one pot") avec un rendement global d'au moins 85 %. Le composé peut ensuite être transformé en 4-méthylamino-4-phénylpipéridine en deux autres étapes qui, elles aussi, peuvent être conduites dans le même réacteur ("one pot").

Ainsi, la présente invention concerne, selon un de ses aspects, un procédé pour la préparation d'une 4-alcoxycarbonylamino-1-benzyl-4-phénylpipéridine de formule (I) 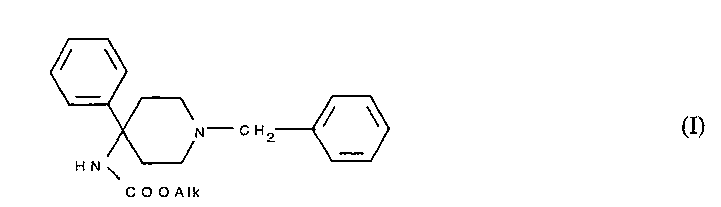 dans laquelle Alk représente un alkyle de 1 à 3 atomes de carbone, caractérisé en ce que l'on hydrolyse en amide la 1-benzyl-4-cyano-4-phénylpipéridine de formule (II) 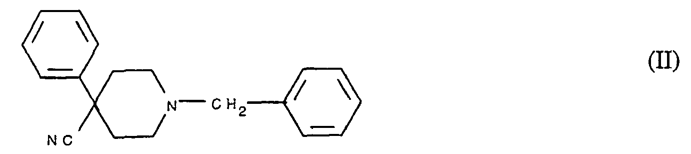 en milieu acide et on traite le 1-benzyl-4-phényl-4-pipéridinecarboxamide ainsi obtenu de formule (III) 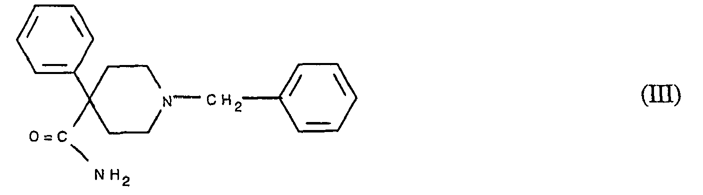 avec du brome en présence d'un alcoolate alcalin de formule (IV)

Alk-OM (IV)

dans laquelle Alk est un (C₁-C₃)alkyle et M est un métal alcalin.

Le composé de départ de formule (II) est un produit commercial disponible sous forme de chlorhydrate, d'où la base peut être facilement libérée par neutralisation avec un hydroxyde alcalin. La 1-benzyl-4-cyano-4-phénylpipéridine peut être préparée *in situ* dans le solvant utilisé pour l'hydrolyse du nitrile en amide.

Cette hydrolyse en amide est conduite de préférence avec de l'acide sulfurique et de l'acide acétique dans un solvant organique, de préférence un hydrocarbure tel que le toluène, à une température de 80-100°C. Après 4-8 heures de chauffage, la réaction est terminée et le 1-benzyl-4-phényl-4-pipéridinecarboxamide (III) est récupéré dans la phase organique et peut être isolé.

Avantageusement, la solution du composé (III) dans le solvant organique, par exemple dans le toluène, est directement utilisée pour la transposition en composé (I). Cette transposition est conduite dans le même solvant par action du brome en présence d'un alcoolate alcalin Alk-OM. Comme alcoolate alcalin on utilise de préférence le méthanolate ou l'éthanolate de sodium ou de potassium (formule IV, Alk=CH₃ ou C₂H₅ et M=Na ou K).

L'addition du brome a lieu à une température de -10 à +10°C, de préférence à 0°C et la réaction se termine après 6-20 heures à la température ambiante.

La 4-alcoxycarbonylamino-1-benzyl-4-phénylpipéridine ainsi obtenue est isolée du mélange réactionnel par addition d'eau, élimination de la phase aqueuse contenant les sous-produits de réaction, notamment des sels inorganiques, et précipitation par addition d'un solvant convenable. Lorsque la transposition a lieu dans le toluène, dans le xylène ou dans le benzène, la précipitation est provoquée par addition d'un hydrocarbure aliphatique ou cycloaliphatique, par exemple l'hexane, le cyclohexane ou le méthylcyclohexane ou dans un éther tel que l'éther isopropylique ou le méthyl-*tert*-butyléther dans lequel le composé (I) est insoluble.

La 4-alcoxycarbonylamino-1-benzyl-4-phénylpipéiidine (I) est obtenue avec un rendement très élevé, calculé à partir du composé (II) de départ, normalement supérieur à 80 % et permet ainsi par exemple la synthèse de la 4-méthylamino-4-phénylpipéridine et de ses sels en seulement 4 étapes dont tant les deux premières que les deux dernières peuvent être conduites dans le même réacteur ("one pot").

Ainsi, selon un autre de ses aspects, la présente invention concerne un procédé pour la préparation de la 4-méthylamino-4-phénylpipéridine, caractérisé en ce que :
(a) on hydrolyse en amide la 1-benzyl-4-cyano-4-phénylpipéridine de formule (II) en milieu acide et on traite la 1-benzyl-4-phényl-4-pipéridinecarboxamide de formule (III) avec du brome et un (C₁-C₃)alcoolate alcalin; puis
(b) on réduit la 4-(C₁-C₃)alcoxycarbonylamino-1-benzyl-4-phénylpipéridine ainsi obtenue avec un hydrure métallique, qui peut être un hydrure mixte, ou avec du Vitride® (hydrure de sodium et d'aluminium bis (2-méthoxyéthoxy) en solution à 70% dans le toluène) et on débenzyle la 1-benzyl-4-méthylamino-4-phénylpipéridine ainsi obtenue, éventuellement sous forme d'un de ses sels, par réduction catalytique et on isole la 4-méthylamino-4-phénylpipéridine ainsi obtenue soit sous forme de base libre soit sous forme d'un de ses sels que l'on transforme éventuellement en base libre.

Selon un mode opératoire très avantageux, ce procédé permet la préparation de la 4-méthylamino-4-phénylpipéridine et de ses sels pharmaceutiquement acceptables par réduction avec l'hydrure de lithium et d'aluminium ou le Vitride® de la 1-benzyl-4-méthoxycarbonylamino-4-phénylpipéridine, par débenzylation soit avec l'acide formique soit sous atmosphère d'hydrogène, en présence de palladium sur charbon ou de palladium sur sulfate de baryum et isolement de la 4-méthylamino-4-phénylpipéridine soit sous forme de base libre, soit sous forme d'un de ses sels, de préférence sous forme de dioxalate, que l'on transforme éventuellement en base libre.

Selon ce mode opératoire très avantageux, la réduction de la 1-benzyl-4-méthoxy (ou éthoxy)carbonylamino-4-phénylpipéridine peut être conduite avec l'hydrure de lithium et d'aluminium dans le tétrahydrofurane, à une température comprise entre 20°C et le reflux du mélange réactionnel. Lorsque la réduction est terminée, l'agent réducteur est détruit selon le méthodes conventionnelles, par exemple par une solution aqueuse d'une base commune : l'hydroxyde de sodium. Après évaporation du tétrahydrofurane, la 1-benzyl-4-méthylamino-4-phénylpipéridine peut être récupérée par évaporation du solvant et isolée ou bien, après évaporation du solvant, elle peut être dissoute dans un solvant du type hydrocarbure, notamment dans du toluène et la solution ainsi obtenue peut être directement utilisée pour la débenzylation.

La réduction de la 1-benzyl-4-méthoxy (ou éthoxy)carbonylamino-4-phénylpipéridine peut être effectuée avec du Vitride®, de préférence dans du toluène à une température de 70 à 105°C, de préférence de 90° à 100°C et, en opérant ensuite comme illustré ci-dessus, la 1-benzyl-4-méthylamino-4-phénylpipéridine est récupérée et isolée ou bien elle peut être soumise directement à la débenzylation dans la solution toluénique de préférence.

Ainsi, à partir du chlorhydrate de 1-cyano-1-benzyl-4-phénylpipéridine, la 4-méthylamino-4-phénylpipéridine peut être obtenue avec un rendement global supérieur à 70 %.

Les meilleurs rendements de préparation de la 4-méthylamino-4-phénylpipéridine sont obtenus par un procédé caractérisé en ce que :
(a') on traite la 1-benzyl-4-cyano-4-phénylpipéridine, préparée *in situ* par neutralisation de son chlorhydrate, avec de l'acide sulfurique à 94-96 % et de l'acide acétique dans le toluène à 80-100°C et on traite le 1-benzyl-4-phényl-4-pipéridinecarboxamide ainsi obtenu avec du brome en présence de méthanolate de sodium ; puis
(b') on réduit la 1-benzyl-4-méthoxycarbonylamino-4-phénylpipéridine ainsi obtenue avec du Vitride® dans le toluène à la température de 90-100°C, on débenzyle par hydrogénolyse, par exemple avec de l'acide formique en présence de palladium la 1-benzyl-4-méthylamino-4-phénylpipéridine ainsi obtenue, ou un de ses sels et on isole la 4-méthylamino-4-phénylpipéridine soit sous forme de base libre, soit sous forme d'un de ses sels qu'on transforme éventuellement en base libre.

Lorsqu'on isole la 4-méthylamino-4-phénylpipéridine sous forme d'un de ses sels, les sels préférés sont le dioxalate et le sesquioxalate éventuellement hydratés.

Les EXEMPLES suivants illustrent l'invention.

### EXEMPLE 1

### Benzyl-4-méthoxycarbonylamino-4-phénylpipéridine

### (a) 1-Benzyl-4-phényl-4-pipéridinecarboxamide.

On chauffe sous azote à 60°C un mélange de 3 kg de chlorhydrate de 1-benzyl-4-cyano-4-phénylpipéridine commercial, 6 l d'eau, 8,98 l de toluène et 1,35 kg d'une solution aqueuse d'hydroxyde de sodium à 35 % et, à cette température, on décante la phase aqueuse. On sèche la phase toluènique par distillation azéotropique et la solution ainsi obtenue contenant la 1-benzyl-4-cyano-4-phénylpipéridine base est ajoutée à un mélange de 2,83 kg d'acide sulfurique à 95 %, 1,73 kg d'acide acétique glacial et 3 l de toluène. On chauffe le mélange ainsi obtenu pendant 6 heures, puis on arrête le chauffage, on refroidit à 60°C et, à cette température on ajoute 3 l d'eau. On laisse revenir à la température ambiante (environ 20°C), puis on verse le mélange dans 8,65 l d'hydroxyde de sodium à 30 % et 6,75 l d'eau. La température monte progressivement à 75°C. On décante à chaud, on lave la phase organique à l'eau et on la sèche par azéotropie. On concentre la solution toluénique de moitié puis on refroidit progressivement à 60°C et on amorce la cristallisation. On termine la cristallisation en refroidissant à 0°C, pendant 2 heures. Le précipité ainsi obtenu est filtré, lavé et séché sous vide à 60 °C. on obtient ainsi 2,68 kg de 1-benzyl-4-phényl-4-pipéridinecarboxamide ; F = 117-118°C.
Pureté : 99 % - Rendement : 95 %.

### (b) 1-Benzyl-4-méthoxycarbonylamino-4-phénylpipéridine.

A une solution de 2,68 kg du produit obtenu à l'étape (a) dans 10,7 l de toluène refroidie à -5°C, on ajoute à cette température, en une heure et sous agitation, une solution de méthanolate de sodium préparée à partir de 4,52 l de méthanol et 532 g de sodium, puis, tout en maintenant la température à environ 0°C, on ajoute 1,85 kg de brome. On laisse le mélange réactionnel pendant 30 minutes à 0°C, puis pendant une nuit à la température ambiante et on lave ensuite à l'eau à 60°C. La solution toluènique est séchée par azéotropie, puis elle est concentrée par distillation de 75 % de la quantité de toluène mise en oeuvre et traitée avec 12 l d'éther isopropylique. On refroidit à 25°C, puis on laisse cristalliser pendant 24 heures à cette température et on refroidit enfin à 0°C. Le produit ainsi précipité est filtré, lavé avec de l'éther isopropylique et séché sous vide à 50°C. On obtient ainsi 2,62 kg de 1-benzyl-4-méthoxycarbonylamino-4-phénylpipéridine avec une pureté HPLC 99 %. F = 121-122°C.

### EXEMPLE 2

### Dioxalate de 4-méthylamino-4-phénylpipéridine

### (a) 1-Benzyl-4-méthoxycarbonylamino-4-phénylpipéridine.

On chauffe sous azote à 60°C un mélange de 3 kg de chlorhydrate de 1-benzyl-4-cyano-4-phénylpipéridine commercial, 61 d'eau, 8,98 l de toluène et 1,35 kg d'une solution aqueuse d'hydroxyde de sodium à 35 % et, à cette température, on décante la phase aqueuse. On sèche la phase toluènique par distillation azéotropique et la solution ainsi obtenue, contenant la 1-benzyl-4-cyano-4-phénylpipéridine base est ajoutée à un mélange de 2,83 kg d'acide sulfurique à 95 %, 1,73 kg d'acide acétique glacial et, 3 l de toluène. On chauffe le mélange ainsi obtenu pendant 6 heures, puis on arrête le chauffage, on refroidit à 60°C et, à cette température on ajoute 3 l d'eau. On laisse revenir à la température ambiante (environ 20°C), puis on verse le mélange dans 8,65 l d'hydroxyde de sodium à 30% et 6,75 l d'eau. La température monte progressivement à 75°C. On décante à chaud, on lave la phase organique à l'eau on la sèche, on la refroidit à -5°C, on ajoute à cette température, en une heure et sous agitation, une solution de méthanolate de sodium préparée à partir de 4,52 l de méthanol et 532 g de sodium, puis, tout en maintenant la température à environ 0°C, on ajoute 1,85 kg de brome. On laisse le mélange réactionnel 30 minutes à 0°C, puis une nuit à la température ambiante et on lave ensuite à l'eau à 60°C. La solution toluènique est séchée par azéotropie, puis elle est concentrée par distillation de 75 % de la quantité de toluène mise en oeuvre et traitée avec 12 l d'éther isopropylique. On refroidit à 25°C, puis on laisse cristalliser 24 heures à la température ambiante et on refroidit enfin à 0°C. Le produit ainsi précipité est filtré, lavé avec de l'éther isopropylique et séché sous vide à 50°C. On obtient ainsi 2,86 kg de 1-benzyl-4-méthoxycarbonylamino-4-phénylpipéridine avec une pureté HPLC 99 %.
Rendement : 92 % calculé à partir du chlorhydrate de 1-benzyl-4-cyano-4-phénylpipéridine.

### (b) Dioxalate de 4-méthylamino-4-phénylpipéridine.

A une solution de 5,54 kg de Vitride® à 70 % dans le toluène, on ajoute 2,89 l de toluène et on chauffe à 98°C. A cette solution, on ajoute en 3 heures à 98-100°C sous agitation, une solution, chauffée à 80°C, de 2,86 kg de la 1-benzyl-4-méthoxycarbonylamino-4-phénylpipéridine, obtenue à l'étape (a), dans 1,3 l de toluène. On ajoute alors à 80°C 3,92 l d'une solution à 5 % d'hydroxyde de sodium. On laisse revenir à 55°C et, à cette température on décante la phase organique et on lave à l'eau. Par extraction de la phase toluènique par 823 g d'acide formique dans 8,64 l d'eau, on obtient 11,77 kg d'une solution de formiate de 1-benzyl-4-méthylamino-4-phénylpipéridine à pH 5,5 qui est ajoutée, à un mélange de 155,2 g de PdBaSO₄ à 5% et 1,35 l d'eau déminéralisée, préalablement chauffée à 85°C sous agitation. On ajoute progressivement, toujours à 85°C, 96,2 g de PdBaSO₄ à 5% et 417,4 g d'acide formique. On chauffe le mélange pendant 12 heures à 85°C, puis on laisse revenir à la température ambiante et on ajoute 898,5 g d'acide sulfurique à 95%. On filtre et on concentre sous vide jusqu'à environ 6,5 l puis on ajoute 6,5 l de 4-méthyl-2-pentanone et on amène le pH du mélange à 14 par addition d'hydroxyde de sodium à 30%. La phase aqueuse est décantée, réextraite avec de la 4-méthyl-2-pentanone et les phases organiques réunies sont séchées par distillation azéotropique. On verse la solution ainsi obtenue dans une solution de 1,57 kg d'acide oxalique dans 13,3 l d'éthanol absolu, on filtre le précipité ainsi obtenu, on le lave à l'éthanol et on le sèche sous vide à 70°C. On obtient ainsi 2,77 kg de dioxalate de 4-méthylamino-4-phénylpipéridine ; F = 214-215°C. Rendement global calculé à partir du chlorhydrate de 1-benzyl-4-cyano-4-phénylpipéridine de départ : 78,5 % de la théorie.

### EXEMPLE 3

### 4-Méthylamino-4-phénylpipéridine base

A une solution de 7,5 g de dioxalate de 4-méthylamino-4-phénylpipéridine dans 30 ml d'eau on ajoute à 20 °C, 6,3 g d'hydroxyde de potassium et 15 ml de 4-méthyl-2-pentanone. On agite, on décante et on écarte la phase aqueuse. On concentre à sec la phase organique et on reprend le résidu huileux avec 15 ml de toluène. La solution toluénique est séchée sur sulfate de magnésium. On filtre les minéraux, on rince le précipité avec 5 ml de toluène et on concentre à sec. On obtient ainsi 3,8 g d'une huile incolore qui est la 4-méthylamino-4-phénylpipéridine base avec une pureté supérieure à 97 %.

### EXEMPLE 4

### Sesquioxalate de 4-méthylamino-4-phénylpipéridine monohydraté

A un mélange de 4,75 l de tétrahydrofurane et de 368,5 g d'hydrure de lithium et d'aluminium, on ajoute, à reflux, une solution de 2,1 kg de 1-benzyl-1-méthoxycarbonylamino-4-phénylpipéridine, obtenue selon l'étape (a) de l'EXEMPLE 2 dans 5 l de tétrahydrofurane. On refroidit à 0°C, puis on ajoute très lentement d'abord 368 ml d'eau dilués dans le tétrahydrofurane, 368 ml d'une solution d'hydroxyde de sodium à 15 % dilués dans le tétrahydrofurane et enfin 736 ml d'eau dilués dans le tétrahydrofurane. On élimine les sels par filtration, on les lave avec du tétrahydrofurane, on réunit les phases organiques, on concentre à sec, on reprend le résidu avec 7 l de toluène, on lave à l'eau et on concentre le toluène à sec. On reprend l'huile avec 12 l d'acétone et on ajoute 583 g d'acide oxalique. On filtre le précipité que l'on met en oeuvre directement dans 14 l de méthanol. On ajoute 280 g de Pd/C à 5 % à 50% d'humidité. On hydrogène à 45°C et à la pression ambiante pendant 24 heures puis on ajoute 3,36 l d'eau au mélange réactionnel et on le chauffe pendant 30 minutes à reflux. A cette température le mélange est filtré, le méthanol est éloigné par distillation et la phase essentiellement aqueuse est chauffée à 95°C. On ajoute 1,4 l de *n*-butanol tout en maintenant la température à 95°C, puis on chauffe à reflux, on refroidit à 20°C, on filtre le précipité, on le lave avec un mélange butanol/eau 9/1 (v/v) et on le sèche à 60°C sous vide. On obtient ainsi 1,34 kg de sesquioxalate de 4-méthylamino-4-phénylpipéridine monohydraté ; F = 252 -254 °C (capillaire)

### EXEMPLE 5

### Dioxalate de 4-méthylamino-4-phénylpipéridine

A une solution de 1,5 kg de 1-benzyl-1-méthoxycarbonylamino-4-phénylpipéridine dans 8 l de toluène chauffée à reflux, on ajoute en une heure 7,01 d'une solution molaire toluénique d'hydrure de lithium et d'aluminium complexé avec 2 moles de tétrahydrofuranne. On maintient à reflux pendant 1 heure puis on refroidit à 30°C et on ajoute très lentement 675 g d'eau. On refroidit à 20°C puis on sépare les insolubles par filtration.

Par extraction de la phase toluénique avec 427,3 g d'acide formique et 4,47 l d'eau, on obtient 6,05 kg d'une solution de formiate de 1-benzyl-4-méthylamino-4-phénylpipéridine qui est ajoutée à un mélange de 81 g de Pd/BaSO₄ à 5% et 0,7 d'eau préalablement chauffée à 85°C sous agitation. On ajoute progressivement, toujours à 85°C, 50,4 g de Pd/BaSO₄ à 5 % et 219 g d'acide formique. On chauffe le mélange pendant 12 heures à 85°C, puis on laisse revenir à la température ambiante et on ajoute 470 g d'acide sulfurique à 95 %. On filtre et on concentre sous vide jusqu'à environ 3,4 puis on ajoute 3,4 l de méthyl-2-pentanone et on amène le pH du mélange à 14 par addition d'hydroxyde de sodium à 30 %. La phase aqueuse est décantée, réextraite avec de la 4-méthyl-2-pentanone et les phases organiques réunies sont séchées par distillation azéotropique. On verse la solution ainsi obtenue dans une solution de 0,82 kg d'acide oxalique dans 7 l d'éthanol absolu, on filtre le précipité ainsi obtenu, on le lave à l'éthanol et on le sèche sous vide à 70°C. On obtient ainsi 1,52 kg de dioxalate de 4-méthylamino-4-phénylpipéridine ; F = 214-215°C.

## Revendications

1. Procédé pour la préparation d'une 4-alcoxycarbonylamino-1-benzyl-4-phénylpipéridine de formule (I) 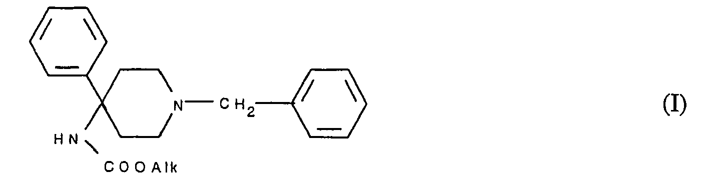 dans laquelle Alk représente un alkyle de 1 à 3 atomes de carbone, **caractérisé en ce que** l'on hydrolyse en amide la 1-benzyl-4-cyano-4-phénylpipéridine de formule (II) 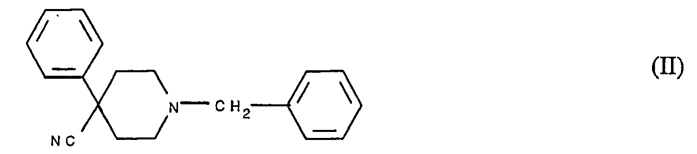 en milieu acide et on traite le 1-benzyl-4-phényl-4-pipéridinecarboxamide ainsi obtenu de formule (III) 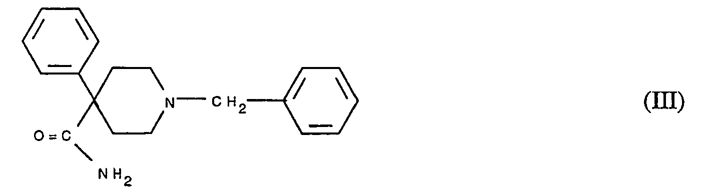 avec du brome en présence d'un alcoolate alcalin de formule (IV)
Alk-OM (IV)
dans laquelle Alk est un (C₁-C₃) alkyle et M est un métal alcalin.

2. Procédé selon la revendication 1, **caractérisé en ce que** l'hydrolyse est conduite avec acide sulfurique et acide acétique dans du toluène à 80-100°C.

3. Procédé selon la revendication 1, **caractérisé en ce que** comme alcoolate alcalin on utilise le méthanolate de sodium ou de potassium ou l'éthanolate de sodium ou de potassium.

4. Procédé pour la préparation de la 4-méthylamino-4-phénylpipéridine et de ses sels, **caractérisé en ce que** :
(a) on hydrolyse en amide la 1-benzyl-4-cyano-4-phénylpipéridine de formule (II) selon la revendication 1 en milieu acide et on traite la 1-benzyl-4-phényl-4-pipéridinecarboxamide de formule (III) selon la revendication 1 avec du brome et un (C₁-C₃) alcoolate alcalin ; puis
(b) on réduit la 4-(C₁-C₃)alcoxycarbonylamino-1-benzyl-4-phénylpipéridine ainsi obtenue avec un hydrure métallique, avec un hydrure metallique mixte, ou avec l'hydrure de sodium et d'aluminium bis(2-méthoxyéthoxy), on débenzyle la 1-benzyl-4-méthylamino-4-phénylpipéridine ainsi obtenue, éventuellement sous forme d'un de ses sels, par réduction catalytique et on isole la 4-méthylamino-4-phénylpipéridine ainsi obtenue soit sous forme de base libre soit sous forme d'un de ses sels que l'on transforme éventuellement en base libre.

5. Procédé selon la revendication 4, **caractérisé en ce que**, dans l'étape (b), la réduction est effectuée avec l'hydrure de lithium et d'aluminium ou avec l'hydrure de sodium et d'aluminium bis(2-méthoxyéthoxy), et la débenzylation est effectuée soit avec l'acide formique soit sous atmosphère d'hydrogène en présence de palladium.

6. Procédé selon la revendication 4, **caractérisé en ce que** :
(a') on traite la 1-benzyl-4-cyano-4-phénylpipéridine, préparée *in situ* par neutralisation de son chlorhydrate, avec de l'acide sulfurique à 94-96 % et de l'acide acétique dans le toluène à 80-100 °C et on traite le 1-benzyl-4-phényl-4-pipéridinecarboxamide ainsi obtenue avec du brome en présence de méthanolate de sodium ; puis
(b') on réduit la 1-benzyl-4-méthoxycarbonylamino-4-phénylpipéridine ainsi obtenue avec l'hydrure de sodium et d'aluminium bis(2-méthoxyéthoxy), dans le toluène à la température de 90-100°C, on débenzyle avec de l'acide formique en présence de palladium la 1-benzyl-4-méthylamino-4-phénylpipéridine ainsi obtenue, ou un de ses sels et on isole la 4-méthylamino-4-phénylpipéridine soit sous forme de base libre, soit sous forme d'un de ses sels qu'on transforme éventuellement en base libre.

## Claims

1. Process for preparing a 4-alkoxycarbonylamino-1-benzyl-4-phenylpiperidine of formula (I) 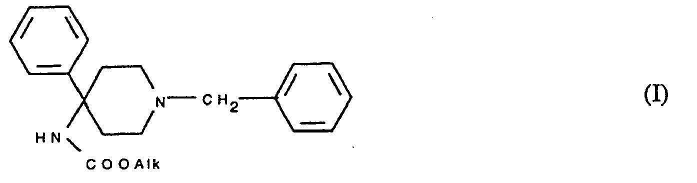 in which Alk represents an alkyl of 1 to 3 carbon atoms, **characterized in that** 1-benzyl-4-cyano-4-phenylpiperidine of formula (II) 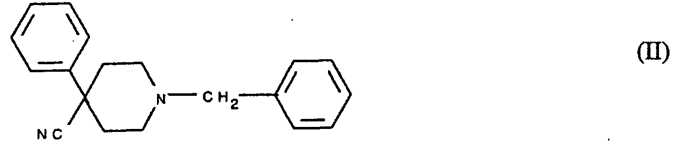 is hydrolysed to the amide in acidic medium and the 1-benzyl-4-phenyl-4-piperidinecarboxamide thus obtained of formula (III) 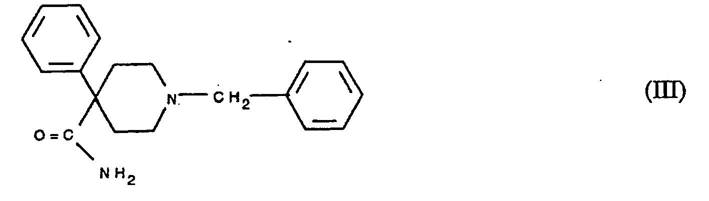 is treated with bromine in the presence of an alkali metal alkoxide of formula (IV)
Alk-OM (IV)
in which Alk is a (C₁-C₃)alkyl and M is an alkali metal.

2. Process according to Claim 1, **characterized in that** the hydrolysis is performed with sulfuric acid and acetic acid in toluene at 80-100°C.

3. Process according to Claim 1, **characterized in that** sodium or potassium methoxide or sodium or potassium ethoxide is used as alkali metal alkoxide.

4. Process for preparing 4-methylamino-4-phenylpiperidine and its salts, **characterized in that**:
(a) 1-benzyl-4-cyano-4-phenylpiperidine of formula (II) according to Claim 1 is hydrolysed to the amide in acidic medium and the 1-benzyl-4-phenyl-4-piperidinecarboxamide of formula (III) according to Claim 1 is treated with bromine and an alkali metal (C₁-C₃) alkoxide; and then
(b) the 4-(C₁-C₃)alkoxycarbonylamino-1-benzyl-4-phenylpiperidine thus obtained is reduced with a metal hydride, with a mixed metal hydride, or with sodium bis(2-methoxyethoxy)aluminium hydride, the 1-benzyl-4-methylamino-4-phenylpiperidine thus obtained, optionally in the form of one of its salts, is debenzylated via catalytic reduction and the 4-methylamino-4-phenylpiperidine thus obtained is isolated either in free base form or in the form of one of its salts and is optionally converted into the free base.

5. Process according to Claim 4, **characterized in that**, in step (b), the reduction is performed with lithium aluminium hydride or sodium bis(2-methoxyethoxy)aluminium hydride, and the debenzylation is performed either with formic acid or under a hydrogen atmosphere in the presence of palladium.

6. Process according to Claim 4, **characterized in that**:
(a') 1-benzyl-4-cyano-4-phenylpiperidine, prepared *in* situ via neutralization of its hydrochloride, is treated with 94-96% sulfuric acid and acetic acid in toluene at 80-100°C and the 1-benzyl-4-phenyl-4-piperidinecarboxamide thus obtained is treated with bromine in the presence of sodium methoxide; and then
(b') the 1-benzyl-4-methoxycarbonylamino-4-phenylpiperidine thus obtained is reduced with sodium bis(2-methoxyethoxy)aluminium hydride in toluene at a temperature of 90-100°C, the 1-benzyl-4-methylamino-4-phenylpiperidine thus obtained, or one of its salts, is debenzylated with formic acid in the presence of palladium, and the 4-methylamino-4-phenylpiperidine is isolated either in the form of the free base or in the form of one of its salts, which is optionally converted into the free base.

## Patentansprüche

1. Verfahren zur Herstellung eines 4-Alkoxycarbonylamino-1-benzyl-4-phenylpiperidins der Formel (I): 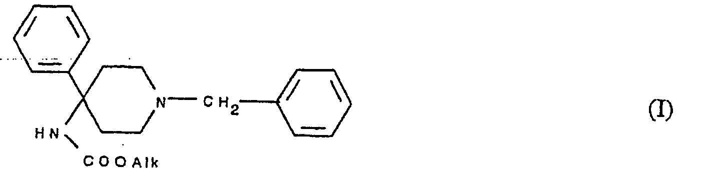 worin Alk für Alkyl mit 1 bis 3 Kohlenstoffatomen steht, **dadurch gekennzeichnet, daß** man 1-Benzyl-4-cyano-4-phenylpiperidin der Formel (II) 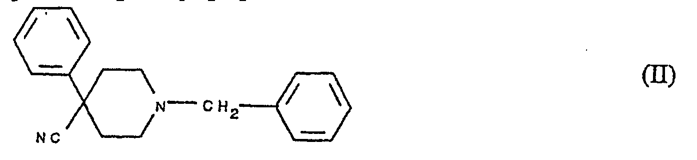 in saurem Milieu zum Amid hydrolysiert und das so erhaltene 1-Benzyl-4-phenyl-4-piperidincarbonsäureamid der Formel (III) 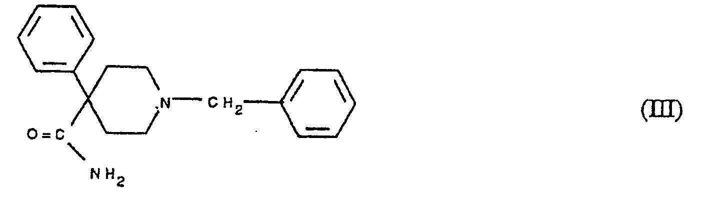 in Gegenwart eines Alkalimetallalkoholats der Formel (IV)
Alk-OM (IV)
worin Alk für (C₁-C₃)-Alkyl steht und M für ein Alkalimetall steht, mit Brom behandelt.

2. Verfahren nach Anspruch 1, **dadurch gekennzeichnet, daß** man die Hydrolyse mit Schwefelsäure und Essigsäure in Toluol bei 80-100°C durchführt.

3. Verfahren nach Anspruch 1, **dadurch gekennzeichnet, daß** man als Alkalimetallalkoholat Natrium- oder Kalium methanolat oder Natrium- oder Kaliumethanolat verwendet.

4. Verfahren zur Herstellung von 4-Methylamino-4-phenylpiperidin und Salzen davon, **dadurch gekennzeichnet, daß** man:
(a) 1-Benzyl-4-cyano-4-phenylpiperidin der Formel (II) gemäß Anspruch 1 in saurem Milieu zum Amid hydrolysiert das so erhaltene 1-Benzyl-4-phenyl-4-piperidincarbonsäureamid der Formel (III) gemäß Anspruch 1 mit Brom und einem (C₁-C₃)-Alkalimetallalkoholat behandelt und dann
(b) das so erhaltene 4-(C₁-C₃)-Alkoxycarbonylamino-1-benzyl-4-phenylpiperidin mit einem Metallhydrid, einem gemischten Metallhydrid oder mit Natriumbis(2-methoxyethoxy)aluminiumhydrid reduziert, das so erhaltene 1-Benzyl-4-methylamino-4-phenylpiperidin, gegebenenfalls in Form eines Salzes davon, durch katalytische Reduktion debenzyliert und das so erhaltene 4-Methylamino-4-phenylpiperidin entweder in Form der freien Base oder in Form eines Salzes davon, welches man gegebenenfalls in die freie Base umwandelt, isoliert.

5. Verfahren nach Anspruch 4, **dadurch gekennzeichnet, daß** man in Schritt (b) die Reduktion mit Lithiumaluminiumhydrid oder mit Natriumbis(2-methoxyethoxy)aluminiumhydrid durchführt und die Debenzylierung entweder mit Ameisensäure oder unter Wasserstoffatmosphäre in Gegenwart von Palladium durchführt.

6. Verfahren nach Anspruch 4, **dadurch gekennzeichnet, daß** man:
(a') in situ durch Neutralisation seines Hydrochlorids hergestelltes 1-Benzyl-4-cyano-4-phenylpiperidin mit 94-96%iger Schwefelsäure und Essigsäure in Toluol bei 80-100°C behandelt und das so erhaltene 1-Benzyl-4-phenyl-4-piperidincarbonsäureamid in Gegenwart von Natriummethanolat mit Brom behandelt und dann
(b') das so erhaltene 1-Benzyl-4-methoxycarbonylamino-4-phenylpiperidin in Toluol bei einer Temperatur von 90-100°C mit Natriumbis (2-methoxyethoxy)aluminiumhydrid reduziert, das so erhaltene 1-Benzyl-4-methylamino-4-phenylpiperidin oder ein Salz davon mit Ameisensäure in Gegenwart von Palladium debenzyliert und das 4-Methylamino-4-phenylpiperidin entweder in Form der freien Base oder in Form eines Salzes davon, welches man gegebenenfalls in die freie Base umwandelt, isoliert.
